# FASCICULE DE BREVET EUROPEEN

(11) **EP 3 341 461 B1**
(45) Date de publication et mention de la délivrance du brevet: **20.12.2023**
(21) Numéro de dépôt: 17733810.0
(22) Date de dépôt: 27.06.2017
(51) Int. Cl.: C12Q 1/04, C11D 3/386, C11D 3/48, C11D 17/04

(54) **SUPPORT IMPRÉGNÉ D'AU MOINS UNE COMPOSITION POUR LE PRÉLÈVEMENT DE MICROORGANISMES PRÉSENTS SUR UNE SURFACE**
MIT MINDESTENS EINER ZUSAMMENSETZUNG IMPRÄGNIERTES SUBSTRAT ZUR PROBENAHME VON MIKROORGANISMEN AUF EINER OBERFLÄCHE
SUBSTRATE IMPREGNATED WITH AT LEAST ONE COMPOSITION FOR THE SAMPLING OF MICROORGANISMS PRESENT ON A SURFACE

(30) Priorité: 29.06.2016 BE 201605499
(43) Date de publication de la demande: 04.07.2018
(60) Demande divisionnaire: 18164313.1
(73) Titulaire: Realco, 1348 Louvain-la-Neuve (BE)
(72) Inventeur: BLACKMAN, Gordon, 1380 LASNES (BE); FASTREZ, Sébastien, 7000 MONS (BE); GATHY, Camille, 1341 CÉROUX-MOUSTY (BE)
(74) Mandataire: Calysta NV
(86) Numéro de dépôt international: PCT/EP2017/065792
(87) Numéro de publication internationale: WO 2018/002013

(56) Documents cités:
- EP-A1- 2 243 821
- EP-A1- 2 537 601
- WO-A1-98/37229
- WO-A1-03/102121
- WO-A1-2004/094080
- WO-A1-2017/037602
- US-A1- 2007 224 249
- "Viscosity of Newtonian and non-Newtonian Fluids", , 21 May 2016 (2016-05-21), Retrieved from the Internet: URL:https://web.archive.org/web/2016052101 2922/https://www.rheosense.com/application s/viscosity/newtonian-non-newtonian [retrieved on 2019-03-15]

## Description

La présente invention se rapporte à un support imprégné d'au moins une composition fluide comprenant au moins un composant enzymatique en solution pour une application sur une surface présentant des microorganismes ayant formé ou non un biofilm.

Par les termes « support imprégné », on entend, au sens de la présente invention, un support dans lequel un fluide se répand ou se diffuse dans toutes ses parties. On peut également parler de support imbibé, les termes « imprégné » et « imbibé » étant utilisés indifféremment au sens de la présente invention.

Dans de nombreux domaines d'activités comme les secteurs agro-alimentaires, les collectivités, les secteurs médicaux et vétérinaires, des contaminations problématiques dues à la présence de microorganismes sont observées très fréquemment. Or, de nos jours, des normes strictes en matière d'hygiène sont imposées et il convient donc de s'assurer que le nombre de microorganismes responsables de telles contaminations soit maintenu sous une valeur seuil acceptable, cette valeur seuil étant propre à chaque domaine d'activité. Classiquement, dans les hôpitaux et dans les cabinets médicaux et vétérinaires, la présence de microorganismes est responsable de maladies nosocomiales tandis que, en agro-alimentaire et en collectivité, ces microorganismes sont responsables de la dégradation de denrées périssables mais aussi du transfert de contaminants vers les consommateurs des produits issus par exemple d'une chaine de production de viandes.

Les bactéries planctoniques, c'est-à-dire les bactéries libres au niveau d'un substrat liquide ou solide, posent en soi un problème puisqu'elles sont à même de contaminer directement tout type de surface comme des denrées alimentaires, des outils médicaux ou encore des patients humains ou des animaux. Toutefois il est aujourd'hui largement reconnu que les problématiques de contaminations par des microorganismes sont d'autant plus importantes que ces derniers forment des biofilms.

Un biofilm est une pellicule visqueuse qui se développe sur toutes les surfaces, suite à l'adhésion de microorganismes sur ces surfaces et à la sécrétion par ceux-ci de polymères qui les recouvrent et facilitent leur adhésion. Les biofilms constituent ainsi une couche de protection autour des microorganismes et représentent une source récurrente de contamination du milieu environnant qui pose des problèmes, par exemple dans les secteurs de l'agro-alimentaire et dans les milieux hospitaliers comme indiqué ci-dessus.

Plus spécifiquement, l'accumulation de polymères sécrétés par les microorganismes de type bactéries crée une matrice composée essentiellement de polysaccharides, d'ADN, de protéines ainsi que de lipides qui protège ces microorganismes des agressions extérieures et qui présente une très forte résistance aux procédures de nettoyage et de désinfection conventionnelles. Les microorganismes se développent donc aisément au sein de cette matrice protectrice et contaminent le milieu environnant en constituant un réservoir particulièrement critique et difficile à éliminer.

Il est reconnu que la problématique de la présence de biofilms est double. Premièrement, comme indiqué plus haut, ceux-ci représentent une source de contamination permanente et très difficile à éliminer par des moyens conventionnels, même les plus agressifs. En effet, les désinfectants classiques sont très souvent inefficaces car il est observé qu'ils ne parviennent pas à atteindre les microorganismes qui sont protégés par la matrice du biofilm composée de polysaccharides, d'ADN, de protéines et de lipides.

Deuxièmement, un biofilm est généralement mixte, en ce sens qu'il est initialement développé par certaines souches bactériennes mais qu'il peut en abriter d'autres, ces souches vivant et se développant en colonies. Or, ces colonies favorisent la communication entre bactéries et, entre autre, l'échange et la propagation de gènes de résistance portés par certaines bactéries. Les biofilms résultant de ces échanges de gènes sont alors encore plus difficiles à éliminer et il faut recourir à des moyens de désinfection ou de traitement de plus en plus puissants qui se heurtent toutefois fréquemment à des problèmes de résistance et/ou de tolérance majeurs.

En milieux hospitalier et vétérinaire mais aussi en milieu agro-alimentaire, la situation est d'autant plus critique que de nombreux microorganismes responsables de la formation de biofilms sont détectés en de nombreux endroits. Dans les hôpitaux et les cabinets vétérinaires, des biofilms sont détectés tant au niveau des individus (patients et animaux) qu'au niveau de l'environnement (salle d'opération, matériel chirurgical, équipements de maintenance de ce matériel, endoscopes, sondes urinaires, cathéters, équipement médical, appareil de dialyse ou de ventilation assistée des individus, ...) et des surfaces (sols, murs, tables d'opération, ...). Dans le secteur de l'agro-alimentaire et notamment dans les usines de fabrication de produits alimentaires, les biofilms peuvent être retrouvés au niveau des machines, des tables, des emballages et des opérateurs même si ces derniers prennent toutes les précautions possibles afin de ne pas contaminer les surfaces et les outils de travail.

De tout ceci, il ressort que les biofilms constituent une réelle problématique, tout particulièrement dans le domaine des soins de santé (hôpitaux, cabinets dentaires...), des soins vétérinaires et de l'agro-alimentaire. Cette problématique est d'autant plus critique que les biofilms peuvent impliquer des bactéries responsables d'infections potentiellement mortelles chez les individus, que ces bactéries soient présentes dans les hôpitaux, les cabinets vétérinaires ou encore dans les produits alimentaires.

Comme indiqué plus haut, la problématique du biofilm est double. D'une part, les désinfectants classiques sont très souvent inefficaces car il est observé qu'ils ne parviennent pas à atteindre les microorganismes qui sont protégés par la matrice du biofilm composée de polysaccharides, d'ADN, de protéines et de lipides. D'autre part, un biofilm étant généralement mixte, il peut contenir plusieurs souches bactériennes favorisant ainsi la propagation de gènes de résistance entre les différentes souches présentes au sein du biofilm et rendant ainsi leur traitement très difficile voir inefficace.

Par conséquent, d'un environnement à l'autre ou d'un secteur d'activité à l'autre, il n'est pas rare et même plutôt fréquent que les biofilms détectés soient totalement différents. Afin de détecter les biofilms, il existe des kits de détection de la présence de biofilms sur des surfaces ou dans des installations plus spécifiques (circuits d'eau,...), ces kits (tel que celui divulgué dans le document EP2537601) permettant de réaliser une coloration des biofilms. Il est donc actuellement possible de déterminer des zones où sont présents des biofilms afin de procéder à une élimination de ces derniers sans toutefois connaître la nature précise des microorganismes (bactéries) à l'origine du biofilm ciblé.

Il en résulte que des compositions d'élimination des biofilms comprenant plusieurs enzymes sont employées sans toutefois savoir si les enzymes utilisées et éventuellement formulées dans une base détergente (voir par exemple le document EP2243821) sont réellement adéquates pour agir sur un biofilm donné. Pour cette raison, les traitements actuels sont plutôt aléatoires et non spécifiques, ce qui est économiquement non rentable.

Par ailleurs, des techniques de prélèvement de microorganismes au départ de surfaces ont été développées afin de caractériser et/ou de quantifier les populations de microorganismes présentes sur des surfaces et responsables de contaminations. Ces méthodes de prélèvements permettent donc de déterminer, sur une surface, les différents types (de souches) de bactéries et de microorganismes présents.

Parmi les méthodes de prélèvement de microorganismes présents sur une surface, l'une des références en la matière est la Norme ISO 18593:2004(F) qui prévoit et définit des méthodes horizontales pour des techniques de prélèvement sur des surfaces (plus spécifiquement sur des surfaces se rencontrant dans l'environnement des industries agro-alimentaires). La méthode à « l'étoffe/éponge » est un exemple de techniques de prélèvement cité dans cette Norme, cette méthode permettant de rechercher et/ou de dénombrer des microorganismes présents sur une surface. Brièvement, selon cette méthode de prélèvement, il s'agit de mouiller l'étoffe/éponge avec une quantité de diluant qui est du sérum physiologique stérile, d'échantillonner la surface dans deux directions perpendiculaires avant d'introduire l'étoffe/éponge dans un récipient stérile avec le diluant. Par la suite, après un éventuel stockage du prélèvement, ce dernier est analysé quantitativement et/ou qualitativement.

Malheureusement, si une telle méthode permet d'assurer un prélèvement des microorganismes libres à la surface d'un substrat, c'est-à-dire un prélèvement de microorganismes planctoniques, elle se révèle être inefficace lorsque la surface est contaminée par des microorganismes y ayant formé un biofilm. En effet, comme expliqué plus haut, la matrice du biofilm composée de polysaccharides, d'ADN, de protéines et de lipides forme une couche polymérique sur les microorganismes, cette matrice constituant une véritable barrière de protection des microorganismes contre des agents extérieurs (chimiques ou/et physiques).

Comme mentionné plus haut, certaines techniques actuelles de prélèvement de microorganismes reposent sur l'utilisation de supports imprégnés d'eau, comme c'est le cas pour les supports décrits dans la Norme ISO 18593:2004(F). Or, le simple fait d'échantillonner une surface dans deux directions perpendiculaires à l'aide par exemple d'une étoffe/éponge imprégnée d'eau physiologique stérile, ne permet pas d'assurer un prélèvement représentatif de l'ensemble des microorganismes contaminant une surface.

Il s'avère en outre que les techniques de prélèvement reposant sur un support imprégné d'eau peuvent au contraire avoir un effet bénéfique sur le développement des microorganismes même suite à la réalisation d'un prélèvement au départ d'une surface. En effet, il est reconnu que certaines bactéries présentent une croissance accrue en présence d'eau, le support imbibé d'eau favorisant donc cette croissance pendant le prélèvement mais aussi après le prélèvement puisqu'une fine pellicule d'eau subsiste sur le support traité où a eu lieu le prélèvement. Ceci peut donc favoriser le développement de certains microorganismes non prélevés par l'étoffe/éponge.

Le document WO98/37229 porte sur une méthode et sur un dispositif de prélèvement de microorganismes au départ d'une surface afin d'en vérifier la propreté, le dispositif comprenant un support imprégné d'une enzyme, à savoir la luciférase. Plus particulièrement, il s'agit d'une tigette dont une des extrémités est constituée d'un matériau absorbant dans lequel est immobilisée de la luciférase et de la luciférine. Par passage de la tigette sur une surface, un échantillon des mircroorganismes présents y est prélévé et, par la suite, l'ATP est détecté selon la réaction de bioluminescence luciférine/luciférase, ce qui permet de déterminer la propreté de la surface en termes de présence de microorganismes.

Malheureusement, avec un tel dispositif de prélèvement, il apparait que les microorganismes ayant formé un biofilm ne sont pas prélevé efficacement. Il en résulte que le prélèvement de microorganimes au départ d'une surface n'est pas représentatif des populations bactériennes y étant pourtant bien présentes.

L'invention a pour but de pallier les inconvénients de l'état de la technique en procurant un support permettant de prélever efficacement, de manière adéquate et représentative, l'ensemble des microorganismes présents sur une surface. En particulier, l'invention a pour but de procurer un support permettant de prélever non seulement les microorganismes libres (planctoniques) mais aussi les microorganismes ayant formé un biofilm sur ladite surface, ceci sans que le procédé de prélèvement utilisant un tel support selon l'invention ne soit plus contraignant ni même forcément plus long (durée d'application du support sur la surface où s'effectue le prélèvement).

L'invention vise donc à procurer un dispositif et un procédé permettant de réaliser un prélèvement simultané des microorganismes libres (planctoniques) et des microorganismes ayant formé un biofilm sur ladite surface, de telle sorte que le prélèvement/échantillonnage soit représentatif de l'ensemble des populations de microorganismes contaminant une surface, ceci n'étant certainement pas le cas lors de prélèvement avec un support imprégné d'eau physiologique ou lors d'un prélèvement réalisé avec un dispositif selon le document WO98/37229.

Pour résoudre ce problème, il est prévu suivant l'invention, un support imprégné tel qu'indiqué au début qui soit destiné à un prélèvement desdits microorganismes sur ladite surface et dont ladite au moins une composition présente une viscosité dynamique comprise entre 50 et 2000 m Pa. s.

Avantageusement, ladite au moins une composition présente une viscosité dynamique comprise entre 100 et 1000 mPa.s, préferentiellement comprise entre 200 et 800 mPa.s, plus préférentiellement comprise entre 300 et 600 mPa.s, encore plus préférentiellement comprise entre 400 et 500 mPa.s.

Dans le cadre de la présente invention, il a été déterminé qu'un support imprégné avec une composition comprenant au moins un composant enzymatique et qui présente une viscosité dynamique comprise entre 50 et 2000 mPa.s permet de prélever/d'échantillonner un nombre significativement plus important de microorganismes, à savoir à la fois les microorganismes libres (bactéries planctoniques) et les microorganismes (bactéries) responsables de la formation de biofilms les protégeant. Au contraire des techniques et des dispositifs de prélèvement classiques tels que décrits plus haut, un support selon l'invention permet donc d'obtenir un échantillonnage réellement représentatif des microorganismes présents sur une surface et non pas seulement un échantillonnage des bactéries libres (planctoniques).

De façon surprenante, il a été déterminé que le fait d'imprégner/d'imbiber un support de type textile (ou tout autre type de support) avec une composition enzymatique selon l'invention permet de prélever des bactéries et d'autres microorganismes dont la présence sur une surface n'était pas attendue ni même soupçonnée. En effet, avec un support selon l'invention, il a été montré :
- que des microorganismes libres sont prélevés de façon au moins équivalente par rapport à ceux prélevés avec les techniques de prélèvement de l'état de la technique (support de prélèvement imprégné d'eau physiologique), et
- que des microorganismes non prélevés avec les supports classiquement utilisés sont prélevés avec un support selon l'invention.

Il en résulte que l'utilisation d'un support selon l'invention, au contraire des supports actuels, permet d'obtenir un prélèvement réellement représentatif et complet des contaminants présents sur une surface. Par conséquent, l'utilisation d'un support selon l'invention permet de pouvoir, par la suite, appliquer un traitement d'élimination des biofilms qui soit ciblé et efficace, ceci en utilisant les composés adéquats et non plus un cocktail de composés dont certains n'ont pas d'intérêt face à une contamination donnée. Ceci représente bien entendu un avantage économique considérable.

L'intérêt d'un support selon l'invention est donc double en ce sens qu'il permet d'échantillonner l'ensemble des contaminants d'une surface et qu'il permet ensuite de déterminer précisément quel traitement d'élimination des microorganismes appliquer. Finalement, avec un support selon l'invention, une élimination adéquate et ciblée de la totalité des microorganismes (planctoniques et protégés par un biofilm) peut être réalisée en aval d'un prélèvement avec ce support imprégné d'une composition enzymatique suivant l'invention.

Selon l'invention, le support de prélèvement permet donc de mettre en évidence, c'est-à-dire de prélever et d'identifier des microorganismes dont la présence n'est pas détectée avec les supports et les méthodes de prélèvement classiques comme la méthode à « l'étoffe/éponge » où le support est imbibé d'eau physiologique ou la méthode selon le document WO98/37229. Par conséquent, l'invention permet véritablement d'optimiser l'échantillonnage et le prélèvement de microorganismes au départ d'une surface, ceci par l'intermédiaire d'un support imprégné d'une composition comprenant au moins un composant enzymatique et présentant une viscosité dynamique comprise entre 50 et 2000 mPa.s, le prélèvement effectué n'étant pas plus contraignant ni même forcément plus long qu'un prélèvement classique.

En particulier, dans le cadre de la présente invention, il a été mis en avant qu'une composition visqueuse selon l'invention, c'est-à-dire qu'une composition comprenant au moins un composant enzymatique et présentant une viscosité dynamique comprise entre 50 et 2000 mPa.s permet de prélever de façon optimale des microorganismes sur des surfaces disposées dans des axes variés. En effet, l'aspect visqueux du support imprégné selon l'invention permet à ce dernier de « coller »/d'adhérer sur la surface et peut, par conséquent être placé sur des surfaces verticales, voire « à l'envers », par exemple sous un plan de travail. L'aspect visqueux du support imprégné selon l'invention permet également de « détacher » les microorganismes et de les « fixer » au niveau du support imprégné de façon optimale : la viscosité dynamique d'une composition selon l'invention permet en effet d'optimiser l'efficacité de prélèvement de microorganismes au départ d'une surface tant sur des surfaces verticales qu'horizontales et permet de garantir que le prélèvement effectué est bien représentatif des populations bactériennes réellement présentes sur les surfaces faisant l'objet du prélèvement.

Par ailleurs, il a été déterminé, dans le cadre de la présente invention, qu'une composition visqueuse et présentant une valeur de viscosité dynamique telle qu'indiquée ci-dessus, permet de réduire significativement le temps de contact nécessaire avec les biofilms présents sur une surface de telle sorte à les déstructurer suffisamment pour en libérer les bactéries et ainsi pouvoir les échantillonner et/ou identifier ces dernières. Il a été montré que le fait que la composition soit visqueuse lui permet véritablement de rester en place et d'englober les biofilms qui se présentent toujours sous forme de fines pellicules correspondant à des « protubérances » de l'ordre de quelques micromètres d'épaisseur, un tel maintien en place n'étant pas aussi optimal avec une composition non visqueuse. En d'autres termes, la viscosité dynamique de la composition comprenant au moins un composant enzymatique au sens de la présente invention permet d'entourer totalement les biofilms et de les déstructurer plus rapidement et plus efficacement, la viscosité de la composition contribuant également à « piéger » les microorganismes (bactéries) prélevés au départ d'une surface. En outre, avec une composition visqueuse selon l'invention, la problématique de l'évaporation rapide de compositions liquides n'est pas rencontrée.

De préférence, selon l'invention, ledit au moins un composant enzymatique comprend au moins une protéase et/ou au moins une laccase et/ou au moins une polysaccharidase. Dans le cadre de la présente invention, il a été mis en évidence que l"utilisation de telles enzymes permet de déstructurer efficacement le biofilm.

Préférentiellement, ledit au moins un composant enzymatique comprend au moins une enzyme additionnelle choisie dans le groupe constitué des oxydo-réductases, des lyases (pectate lyase , ...), des transférases, des peptidases (métallo-protéase, sérine-protéases, exo-peptidase, endo-protéase, cystine-protéase, ...), des lipases et des estérases (lysophospholipase, phospholipase, ...) et des glucosam inidases.

De préférence, selon l'invention, ledit support imprégné est une lingette ou une éponge ou une étoffe ou tout autre type de support adéquat. Dans le cadre de la présente invention les supports peuvent être imprégnés d'au moins une composition suivant l'invention et permettent un attachement des microorganismes au niveau du support.

Avantageusement, selon l'invention, ladite au moins une composition comprenant au moins un composant enzymatique présente un pH compris entre 5 et 11.

Dans une forme de réalisation particulièrement avantageuse du dispositif selon l'invention, ledit support et ladite au moins une composition comprenant au moins un composant enzymatique sont stériles. En effet, il est avantageux que ledit support imprégné de même que ladite au moins composition comprenant au moins un composant enzymatique soient stériles afin que d'autres contaminants extérieurs ne viennent pas fausser l'analyse des microorganismes prélevés tant d'un point de vue qualitatif que quantitatif, l'objectif étant de réaliser un prélèvement/échantillonnage reflétant fidèlement les contaminations en microorganismes d'une surface.

Avantageusement, selon l'invention, ladite au moins une composition comprenant au moins un composant enzymatique comprend en outre au moins un composant détergent.

De préférence, selon l'invention, ledit au moins un composant détergent comprend au moins un agent mouillant et/ou au moins un agent séquestrant et/ou au moins un agent dispersant.

L'agent mouillant du composant détergent est une substance chimique amphiphile, ou une composition comprenant ladite substance chimique amphiphile, qui modifie la tension superficielle entre deux surfaces. L'agent mouillant a pour avantage de favoriser l'étalement d'un liquide sur un solide mais aussi d'accentuer le contact entre deux surfaces. Plus particulièrement, l'agent mouillant favorise un contact entre le composant détergent et une surface et, par conséquent, entre les enzymes et leur substrat. Par exemple, sur des surfaces en inox fréquemment rencontrées dans les industries agro-alimentaires mais aussi en milieu hospitalier ou vétérinaire, l'agent mouillant permet de réaliser un étalement homogène de la composition et ainsi sa répartition parfaite sur les surfaces à décontaminer, par exemple sur les outils de production, les plans de travail, les sols ou encore les tables d'opérations et les outils médicaux.

L'agent mouillant peut être anionique, cationique, non-ionique ou zwitterionique. Préférentiellement, l'agent mouillant peut être un mouillant anionique ou non-ionique, c'est-à-dire que la partie hydrophile est chargée négativement ou ne comporte aucune charge nette, ou peut être une composition comprenant un mouillant anionique. Plus particulièrement, l'agent mouillant peut être un ester de saccharose ou une composition comprenant un alkyle sulfate de sodium et un alcool.

L'agent séquestrant est une substance chimique ayant la capacité à former des complexes avec des ions minéraux qu'il fixe sous une forme empêchant leur précipitation par les réactions habituelles. A titre d'exemple, l'agent séquestrant peut être l'acide éthylène-diamine-tétraacétique, le glucono-delta-lactone, le gluconate de sodium, le gluconate de potassium, le gluconate de calcium, l'acide citrique, l'acide phosphorique, l'acide tartrique, l'acétate de sodium, le sorbitol, un composé comportant un atome de phosphore. Préférentiellement, l'agent séquestrant peut être un oxyde de phosphore tel qu'un phosphonate, un phosphinate ou un phosphate ou leur mélange, ou un sel de celui-ci, une amine ou un oxyde d'amine portant au moins, dans sa structure, un groupement fonctionnel phosphine, oxyde de phosphine, phophinite, phosphonite, phosphite, phosphonate, phosphinate ou phosphate, seul ou en combinaison, ou un sel de ceux-ci.

Plus préférentiellement, l'agent séquestrant peut être un phosphonate ou un sel de celui-ci, une amine ou un oxyde d'amine comportant au moins, dans sa structure, un groupement fonctionnel phosphine, oxyde de phosphine, phosphinite, phosphonite, phosphite, phosphonate, phosphinate ou phosphate, seul ou en combinaison, ou un sel de ceux-ci. A titre d'exemple non limitatif, le phosphonate peut être de formule générale R¹(R²O)(R³O)P=O dans lequel R¹, R² et R³ représentent indépendamment un groupe hydrogène, alkyle, alkyle substitué, alkyle-amino substitué ou non, aminoalkyl substitué ou non, aryle ou aryle substitué. A titre d'exemple non limitatif, l'amine ou l'oxyde d'amine peuvent comporter un, deux ou trois substituant(s) de formule générale CR⁴R⁵W dans laquelle R⁴ et R⁵ représentent indépendamment l'un de l'autre un groupe hydrogène, alkyle, alkyle substitué, alkyle-amino substitué ou non, aminoalkyl substitué ou non, aryle ou aryle substitué, et W représente un groupe phosphonate, phosphinate ou phosphate.

L'agent séquestrant peut être sous la forme d'un sel de sodium, calcium, lithium, magnésium ou potassium ; préférentiellement, l'agent séquestrant peut être sous la forme d'un sel de sodium, calcium ou potassium.

De préférence, l'agent séquestrant est un agent qui peut être utilisé sans danger dans le secteur alimentaire, c'est-à-dire que l'agent séquestrant ne présente aucun danger pour la santé, seul ou en association avec d'autres composants.

L'agent dispersant du composant détergent permet d'améliorer la séparation des particules d'une suspension afin de prévenir l'agglutination, l'agrégation et/ou la décantation. Cet agent dispersant peut être un polymère soluble ou partiellement soluble dans l'eau tel que par exemple le polyéthylène glycol, les dérivés de la cellulose ou un polymère comprenant au moins un motif acide acrylique ou ester acrylique. Préférentiellement, l'agent dispersant est un polymère comprenant au moins un motif acide acrylique ou ester acrylique de formule générale -(CH₂-CH-COOR)- dans lequel R représente un groupe hydrogène, alkyle ou alkyle substitué, aryle ou aryle substitué. Par exemple, l'agent dispersant est un polymère ayant un poids moléculaire moyen Mw compris approximativement entre 500 et 10000.

Plus préférentiellement, l'agent dispersant est un polymère de l'acide acrylique. Par exemple, l'agent dispersant peut être un homopolymère de l'acide acrylique ayant un poids moléculaire moyen compris approximativement entre 2000 et 6000.

La présence d'un agent dispersant dans la composition suivant l'invention permet donc d'éviter toute agrégation de particules bactériennes lors du nettoyage de surfaces, ce qui assure une élimination optimale des particules de biofilms détachées d'un support sous l'action des enzymes. En effet, plutôt que de s'agréger, ces particules restent séparées dans une suspension, ne se redéposent pas et ne ré-adhèrent pas sur le support nettoyé.

De préférence, selon l'invention, ledit au moins un composant détergent comprend une proportion d'agent séquestrant comprise entre 1 et 10%, une proportion d'agent dispersant comprise entre 1 et 10% et une proportion d'agent mouillant comprise entre 1 et 30% en poids par rapport au poids total dudit composant détergent. De préférence, ledit au moins un composant détergent comprend une proportion d'agent mouillant comprise entre 5 et 20%, préférentiellement une proportion d'agent mouillant égale à 15% en poids par rapport au poids total dudit au moins un composant détergent.

D'autres formes de réalisation d'un support imprégné d'au moins une composition sont indiquées dans les revendications annexées.

L'invention porte également sur un procédé de prélèvement de microorganismes présents sur une surface, en particulier de prélèvement de microorganismes ayant formé ou non un biofilm sur ladite surface, à l'aide d'un support imprégné d'au moins une composition fluide selon l'invention, ledit procédé comprenant les étapes suivantes :
- une application dudit support imprégné sur ladite surface pendant une période de temps prédéterminée afin de prélever, au départ de ladite surface, des microorganismes y ayant formé ou non un biofilm ; et
- un retrait dudit support de ladite surface.

L'imprégnation du support selon l'invention peut se faire préalablement à une application du support sur une surface ou suite à une application du support non encore imprégné sur une surface.

Avantageusement, selon l'invention, ladite période de temps prédéterminée d'application dudit support imprégné sur ladite surface est comprise entre 1 et 45 minutes et est de préférence de 15 minutes.

Dans une forme de réalisation particulière, ledit procédé selon l'invention comprend une étape préalable de chauffage de ladite au moins une composition comprenant ledit au moins un composant enzymatique à une température comprise entre 15 et 65°C, de préférence à une température égale à 45°. Cette étape de chauffage de l'enzyme permet d'activer cette dernière et d'obtenir 100% d'activité enzymatique.

De préférence, selon l'invention, ledit procédé comprend une étape additionnelle d'écouvillonnage de ladite surface de telle sorte que des microorganismes non prélevés par ledit support imprégné soit prélevés à l'aide d'écouvillons. Un tel prélèvement additionnel renforce le fait que le prélèvement/l'échantillonnage effectué reflète véritablement les contaminations en microorganismes sur une surface ou un substrat. De façon alternative, plutôt que de réaliser un écouvillonage, un support substantiellement identique au support imprégné suivant l'invention mais non imprégné peut être utilisé.

Avantageusement, selon l'invention, ledit procédé comprend une étape additionnelle d'extraction desdits microorganismes prélevés dans une solution d'extraction stérile.

Dans une forme de réalisation particulièrement avantageuse du procédé selon l'invention, ledit procédé comprend une étape additionnelle de quantification et/ou d'identification desdits microorganismes extraits.

D'autres formes de réalisation du procédé de prélèvement de microorganismes présents sur une surface suivant l'invention sont indiquées dans les revendications annexées.

L'invention porte également sur une trousse pour le prélèvement de microorganismes présents sur une surface, en particulier pour le prélèvement de microorganismes ayant formé ou non un biofilm sur ladite surface, ladite trousse comprenant :
- un support, par exemple un support de type textile ; et
- un échantillon d'au moins un composant enzymatique en solution ou sous forme solide à mettre en solution avant l'imprégnation du support.

L'échantillon d'au moins un composant enzymatique présente, en solution, une viscosité dynamique comprise entre 50 et 2000 mPa.s. Lorsque l'échantillon d'au moins un composant enzymatique est initialement sous forme solide, une mise en solution de cette dernière est effectuée pour obtenir un composant enzymatique présentant une viscosité dynamique comprise entre 50 et 2000 mPa.s.

Avantageusement, selon l'invention, ladite trousse comprend en outre un échantillon d'au moins un composant détergent. Bien évidemment, la trousse selon l'invention peut présenter un échantillon comprenant ledit au moins composant enzymatique et ledit au moins un composant détergent formulés ensemble ou séparément en solution ou sous forme solide. Lorsque l'échantillon se présente sous une forme solide, une dilution/mise en solution est réalisée avant l'imprégnation/l'imbibition du support.

De préférence, selon l'invention, ledit échantillon d'au moins un détergent comprend au moins un agent mouillant et/ou au moins un agent séquestrant et/ou au moins un agent dispersant.

De plus, dans une forme de réalisation particulière, ledit un échantillon d'au moins un composant enzymatique comprend au moins une protéase et/ou au moins une laccase et/ou au moins une polysaccharidase.

D'autres formes de réalisation d'une trousse pour le prélèvement de microorganismes présents sur une surface, en particulier pour le prélèvement de microorganismes ayant formé ou non un biofilm sur ladite surface sont indiquées dans les revendications annexées.

La présente invention porte également sur une utilisation d'un support imprégné selon l'invention pour le prélèvement de microorganismes présents sur une surface, en particulier de microorganismes ayant formé ou non un biofilm sur ladite surface.

La présente invention porte également sur une utilisation d'une trousse selon l'invention pour le prélèvement de microorganismes présents sur une surface, en particulier de microorganismes ayant formé ou non un biofilm sur ladite surface.

D'autres formes d'utilisation d'un support imprégné ou d'une trousse selon l'invention pour le prélèvement de microorganismes présents sur une surface, en particulier pour le prélèvement de microorganismes ayant formé ou non un biofilm sur ladite surface sont indiquées dans les revendications annexées.

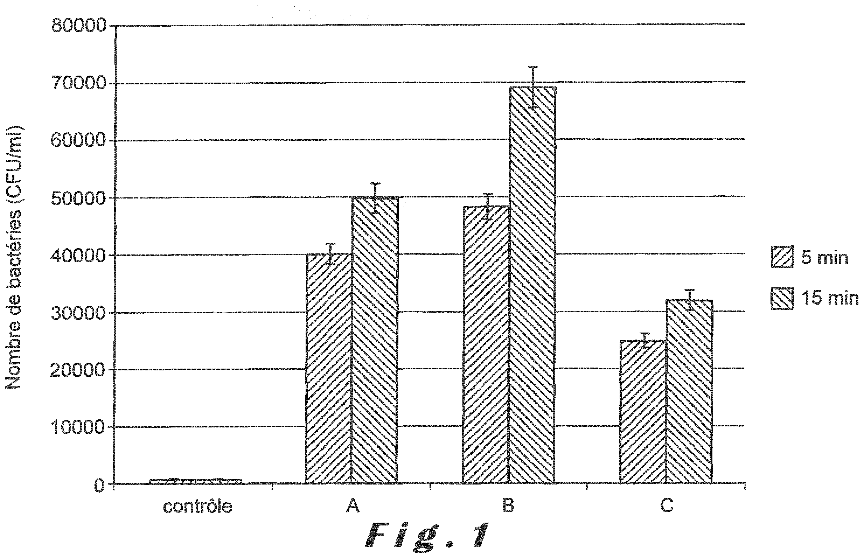

La figure 1 illustre les résultats (bactérie CFU/ml) obtenus lors d'analyses ATPmétriques de solutions obtenues par extraction au départ de supports imprégnés avec des compositions présentant différentes viscosités dynamiques et ayant été appliqués durant 5 ou 15 min sur une surface en inox comprenant du biofilm formé par *Staphylococcus epidermidis* et une souillure protéique (BSA).

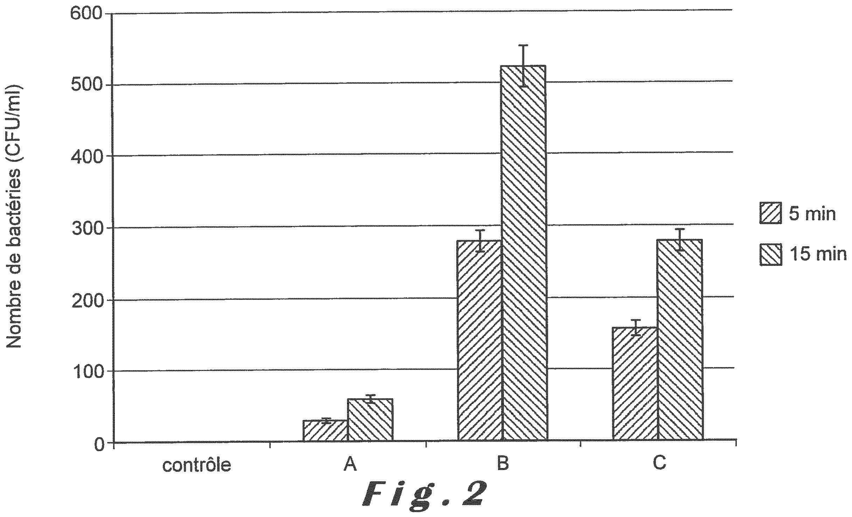

La figure 2 illustre les résultats (bactérie CFU/ml) obtenus lors du dénombrement des colonies bactériennes développées au départ de solutions obtenues par extraction à partir de supports imprégnés avec des compositions présentant différentes viscosités dynamiques et ayant été appliqués durant 5 ou 15 min sur une surface en inox comprenant du biofilm formé par *Staphylococcus epidermidis* et une souillure protéique (BSA).

### Exemples

### Exemple 1 : Efficacité de prélèvement au départ d'une surface de microorganismes protégés par un biofilm selon que le support selon l'invention est imprégné d'une composition dont la viscosité dynamique est différente

Différentes viscosités dynamiques de compositions imprégnant un support selon l'invention pour le prélèvement de microorganismes présents sur une surface, en particulier pour le prélèvement de microorganismes ayant formé un biofilm sur cette surface, ont été testées en termes d'efficacité de prélèvement des microorganismes au départ de cette surface. Pour se faire, les protocoles suivant ont été respectés.

### a) Protocole pour le développement de biofilm sur des surfaces en inox

**Jour-1 :** Inoculation d'une culture de *Staphylococcus epidermidis* dans un erlenmeyer contenant 20 mL de milieu TSB (Tryptic Soy Broth) stérile avec maintien sous agitation (120 RPM) à 35 °C pendant une nuit.
**Jour 0 :**
   - placement de surfaces stériles en inox dans une boite de pétri stérile avec traçage d'une ligne hydrophobe sur le contour des surfaces afin de permettre un maintien de la souillure de BSA sur la surface (voir ci-dessous : protocole de dépôt de souillure BSA) ;
   - ajout de milieu TSB jusqu'à recouvrir les surfaces en inox ;
   - prélèvement d'1 mL de la pré-culture de *Staphylococcus epidermidis* préparée la veille et placement de ce prélèvement dans la boite de pétri ;
   - agitation selon un mouvement rotatif de la boite de pétri puis incubation de cette dernière durant au moins 20h à 35°C.
**Jour + 1 :**
   - évacuation du milieu de culture et rinçage des surfaces avec 20 mL d'eau adoucie stérile à deux reprises ;
   - séchage des surfaces à une température de 40 °C pendant au moins 1 heure.

### b) Protocole de dépôt de souillure BSA (Bovin Sérum Albumin)

Une couche de souillure protéique a été ajouté sur la couche de biofilm formé selon le protocole du point a) ci-dessus, ceci permettant de simuler la situation réelle où du biofilm peut être très fréquemment associé à des souillures résiduelles. Une solution contenant 10 mg/mL de BSA a été préparée avant application de 7 ml de solution sur chaque surface et un séchage subséquent des surfaces à une température de 40°C maximum pendant quelques heures.

### c) Protocole de prélèvement des contaminants présents sur les surfaces en inox

Des supports en tissu stérile (Kaliwipe premium KW-P8030 du fournisseur Conformat) ont été découpés afin d'être de la même dimension que les surface à analyser, à savoir 10x10 cm, et ont été imbibés avec l'une des compositions suivante préalablement chauffée à 45°C :
A) une solution aqueuse contenant : laureth sulfate de sodium (agent détergent) à raison de 5,4% en volume, lauramine oxyde (agent mouillant) à raison de 3% en volume, carboxyméthylinuline sel de sodium (séquestrant) à raison de 0,11% en volume, protéases à raison de 0,31% en volume (Savinase 16L type EX et Alcalase 2,5L type DX), laccase à raison de 0,005% en volume (Novozym 51003), lipase à raison de 0,07% en volume (Lipolase 100L), alpha-amylase à raison de 0,036% en volume (Amplify 12L), cellulase à raison de 0,017% en volume (Carezyme 4500L) et mannanase à raison de 0,005% en volume (Mannaway 4.0L) : viscosité dynamique de 8,4 mPa.s ;
B) une solution aqueuse contenant : laureth sulfate de sodium (agent détergent) à raison de 5,4% en volume, chlorure de sodium (permettant de viscoser la solution) à raison de 1% en volume, lauramine oxyde (agent mouillant) à raison de 3% en volume, carboxyméthylinuline sel de sodium (séquestrant) à raison de 0,11 % en volume, protéases à raison de 0,31% en volume volume (Savinase 16L type EX et Alcalase 2,5L type DX), laccase à raison de 0,005% en volume (Novozym 51003), lipase à raison de 0,07% en volume (Lipolase 100L), alpha-amylase à raison de 0,036% en volume (Amplify 12L), cellulase à raison de 0,017% en volume (Carezyme 4500L)et mannanase à raison de 0,005% en volume (Mannaway 4.0L) : viscosité dynamique de 396,5 mPa.s ;
C) une solution aqueuse contenant : laureth sulfate de sodium (agent détergent) à raison de 5,4% en volume, chlorure de sodium (permettant de viscoser la solution) à raison de 1,2 % en volume, lauramine oxyde (agent mouillant) à raison de 3% en volume, carboxyméthylinuline sel de sodium (séquestrant) à raison de 0,11 % en volume, protéases à raison de 0,31% en volume volume (Savinase 16L type EX et Alcalase 2,5L type DX), laccase à raison de 0,005% en volume (Novozym 51003), lipase à raison de 0,07% en volume (Lipolase 100L), alpha-amylase à raison de 0,036% en volume (Amplify 12L), cellulase à raison de 0,017% en volume (Carezyme 4500L)et mannanase à raison de 0,005% en volume (Mannaway 4.0L) : viscosité dynamique de 2213 mPa.s.
Chacun de ces supports imprégnés a ensuite été posé sur une surface en inox verticale obtenue à l'issue des protocoles a) et b) ci-dessus. Après le temps de contact de 5 ou 15 min, les tissus ont été prélevés (récupérés) stérilement. Ensuite, durant 30 secondes, un tissu stérile sec non imbibé a été appliqué sur les surfaces afin d'absorber la solution de prélèvement y étant encore présente. Pour une surface en inox donnée, les tissus imbibé et non imbibé ont ensuite été regroupés dans un sachet stérile.

### d) Protocole d'extraction des échantillons prélevés

Afin d'extraire, au départ des tissus issus du protocole sous le point c) ci-dessus, les molécules (contaminants) prélevées au départ des surfaces en inox, 90 mL de la solution Thio ont été ajoutés dans le sachet stérile avant son passage au malaxeur pendant 2 minutes afin d'obtenir une solution prête à être analysée. La solution Thio est une solution stérile contenant plusieurs composants ayant comme fonction la neutralisation de l'effet biocide des produits qui aurait pu être utilisés sur les surfaces (désinfectants utilisés lors des phases de nettoyage/ désinfection en industrie par exemple). Cette solution comprend : polysorbate 80 3g/L, lécithine 0,3g/L, L-histidine 0,1g/L, thiosulfate de sodium 0,5g/L. Ces composant sont dilués dans de l'eau déminéralisée et le volume est porté à 1L. La solution est stérilisée dans un autoclave avant utilisation.

### e) Analyses des solutions

Deux types d'analyses ont été réalisées :

### 1. ATP-métrie de seconde génération (ATP-2G)

L'analyse des solutions obtenues après extraction a été réalisée selon la technique ATP-2G qui est une technologie permettant de mesurer la quantité d'ATP intracellulaire dans un échantillons en quelques minutes. Ce dosage quantifie tous les micro-organismes vivants en milieu liquide. Cette mesure a été réalisée avec le kit QGA 2G commercialisé par la société Aqua-tools^{®} en respectant les consignes et le protocole du fabriquant qui indique comment convertir la mesure ATP en nombre de colonies.

Les résultats obtenus sont présentés à la figure 1. Comme on peut le constater, l'analyse ATP-métrique montre un niveau de contamination plus important pour la solution ayant une viscosité dynamique comprises entre 50 et 2000 mPa.s que pour les solutions ayant des viscosités plus basses ou plus élevées. Ceci signifie que, suivant une viscosité dynamique comprises entre 50 et 2000 mPa.s de la composition imprégnant le support de prélèvement selon l'invention, un prélèvement de l'ensemble des contaminants bactériens et autre est effectué. Du moins, un prélèvement optimisé est réalisé avec un support de prélèvement imprégné d'une composition présentant une telle viscosité dynamique suivant l'invention.

### 2. Comptage bactérien

Les solutions obtenues à l'issue du protocole sous le point d) ci-dessus ont également été étalées sur boîte de pétri contenant du milieu PCA généraliste non spécifique (n = 3), les boites étant ensuite incubées à 35°C durant 24h avant dénombrement des colonies qui s'y sont développées.

Les résultats obtenus sont présentés à la figure 2. Comme on peut le constater, la même tendance que celle observée pour l'ATP-métrie se marque, à savoir qu'une contamination des solutions plus importante est observée pour les solutions présentant une viscosité dynamique comprise entre 50 et 2000 mPa.s. Les niveaux de contamination détectés sont beaucoup plus faibles que pour l'ATP-métrie, cela s'expliquant par le fait que les microorganismes du biofilm sont peu cultivables sur boîte de pétri (état de stress qui les rend indétectables par cette technique microbiologique classique).

Ceci signifie à nouveau qu'avec une telle viscosité dynamique de la composition imprégnant le support de prélèvement selon l'invention, un prélèvement de l'ensemble des contaminants bactériens et autre est effectué. Du moins, un prélèvement optimisé est réalisé avec un support de prélèvement imprégné d'une composition présentant une telle viscosité dynamique suivant l'invention.

### Exemple 2 : Adhérence d'un support de prélèvement selon l'invention sur des surfaces verticales et horizontales selon la viscosité dynamique de la composition imprégnée dans ce support

Le protocole de prélèvement tel que celui décrit à l'exemple 1 est appliqué avec les trois solutions (A, B et C) de viscosités différentes. Pour chaque solution (composition), un essai est réalisé sur une surface verticale et sous une surface horizontale puisque le fait de pouvoir prélever sous une surface peut s'avérer être particulièrement intéressant en industrie.

Plusieurs paramètres ont été relevés suite à l'application des supports de prélèvement :
- imbibation du tissu par la composition de prélèvement et répartition de celles-ci ;
- tenue des tissus sur la surface ;
- écoulement de la composition de prélèvement hors du tissu sur la surface ; et
- répartition de la composition de prélèvement sur la surface lorsque le tissu est retiré de la surface : la surface devrait de préférence être entièrement recouverte d'une fine couche de composition de prélèvement lorsque le premier tissu est retiré.

Le Tableau 1 ci-dessous reprend les résultats obtenus pour ces différents paramètres.

**Tableau 1**

| **Solution** | **Sur une surface verticale** | **Sous une surface horizontale** |
|---|---|---|
| **A :** | - bonne répartition de la composition de prélèvement sur le tissu et sur la surface. | - bonne répartition de la composition de prélèvement sur le tissu et sur la surface. |
| viscosité de 8,4 mPa.s | | |
| | - le tissu reste collé pendant 15 min mais écoulement de la composition après 1 min sur une longueur de plus de 22 cm. | - le tissu reste collé pendant 15 min, pas d'écoulement. |
| **B :** | - bonne répartition de la composition de prélèvement sur le tissu et sur la surface. | - bonne répartition de la composition de prélèvement sur le tissu et sur la surface. |
| viscosité de 396,5 mPa.s | | |
| | - le tissu reste collé pendant 15 min, pas d'écoulement et reste bien collé àla surface. | - le tissu reste collé pendant 15 min, pas d'écoulement et reste bien collé à la surface. |
| | - après retrait du tissu, 100 % de la surface est couverte de composition. | - après retrait du tissu, 100 % de la surface est couverte de composition. |
| **C :** | - mauvaise répartition de la composition de prélèvement sur le tissu et sur la surface, une partie du tissus reste sec. | - mauvaise répartition de la composition de prélèvement sur le tissu et sur la surface, une partie du tissus reste sec. |
| viscosité de 2.213 mPa.s | | |
| | - après retrait du tissu, une partie de la surface n'est pas couverte (90 % de surface couverte). | - après retrait du tissu, une partie de la surface n'est pas couverte (90 % de surface couverte). |
| | - le tissu reste collé pendant 15 min, pas d'écoulement. | - le tissus se décolle sur les coin. |

Une comparaison des différents paramètres relevés permet de constater que ce sont les support de prélèvement selon l'invention imprégnés avec une composition présentant une viscosité dynamique comprise entre 50 et 2000 mPa.s qui possèdent les meilleurs caractéristiques permettant de garantir que le prélèvement sera adéquat et bien représentatif des populations de microorganismes présentes sur les surfaces.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Support imprégné d'au moins une composition fluide comprenant au moins un composant enzymatique en solution pour une application sur une surface présentant des microorganismes ayant formé ou non un biofilm, **caractérisé en ce que** ledit support imprégné est destiné à un prélèvement desdits microorganismes sur ladite surface et **en ce que** ladite au moins une composition présente une viscosité dynamique comprise entre 50 et 2000 mPa.s.

2. Support selon la revendication 1, **caractérisé en ce que** ledit au moins un composant enzymatique comprend au moins une protéase et/ou au moins une laccase et/ou au moins une polysaccharidase.

3. Support selon la revendication 1 ou 2, caractérisé en ce ledit support est une lingette ou une éponge ou une étoffe ou tout autre type de support adéquat.

4. Support selon l'une quelconque des revendications 1 à 3, **caractérisé en ce que** ladite au moins une composition comprenant au moins un composant enzymatique présente un pH compris entre 5 et 11.

5. Support selon l'une quelconque des revendications 1 à 4, caractérisé en ce ledit support et ladite au moins une composition comprenant au moins un composant enzymatique sont stériles.

6. Support selon l'une quelconque de revendications 1 à 5, **caractérisé en ce que** ladite au moins une composition comprenant au moins un composant enzymatique comprend en outre au moins un composant détergent.

7. Support selon la revendication 6, **caractérisé en ce que** ledit au moins un composant détergent comprend au moins un agent mouillant et/ou au moins un agent séquestrant et/ou au moins un agent dispersant.

8. Support selon les revendications 6 ou 7, **caractérisé en ce que** ledit au moins un composant détergent comprend une proportion d'agent séquestrant comprise entre 1 et 10%, une proportion d'agent dispersant comprise entre 1 et 10% et une proportion d'agent mouillant comprise entre 1 et 30% en poids par rapport au poids total dudit au moins un composant détergent.

9. Procédé de prélèvement de microorganismes présents sur une surface, en particulier de prélèvement de microorganismes ayant formé ou non un biofilm sur ladite surface, à l'aide d'un support imprégné d'au moins une composition fluide selon l'une quelconque des revendications 1 à 8, ledit procédé comprenant les étapes suivantes :
- une application dudit support imprégné sur ladite surface pendant une période de temps prédéterminée afin de prélever, au départ de ladite surface, des microorganismes y ayant formé ou non un biofilm; et
- un retrait dudit support de ladite surface.

10. Procédé selon la revendication 9, **caractérisé en ce que** ladite période de temps prédéterminée d'application dudit support imprégné sur ladite surface est comprise entre 1 et 45 minutes et est de préférence de 15 minutes.

11. Procédé selon les revendications 9 ou 10, **caractérisé en ce qu'**il comprend une étape préalable de chauffage de ladite au moins une composition comprenant ledit au moins un composant enzymatique à une température comprise entre 15 et 65°C, de préférence à une température égale à 45°C.

12. Procédé selon l'une quelconque des revendications 9 à 11, **caractérisé en ce qu'**il comprend une étape additionnelle d'écouvillonnage de ladite surface de telle sorte que des microorganismes non prélevés par ledit support imprégné soit prélevés à l'aide d'écouvillons.

13. Procédé selon l'une quelconque des revendications 9 à 12, **caractérisé en ce qu'**il comprend une étape additionnelle d'extraction desdits microorganismes prélevés dans une solution d'extraction stérile.

14. Procédé selon la revendication 13, **caractérisé en ce qu'**il comprend une étape additionnelle de quantification et/ou d'identification desdits microorganismes extraits.

15. Trousse pour la préparation d'un support imprégné d'au moins une composition fluide selon l'une quelconque des revendications 1 à 8, ladite trousse comprenant :
- un support, par exemple un support de type textile; et
- un échantillon d'au moins un composant enzymatique en solution.

16. Trousse pour la préparation d'un support imprégné d'au moins une composition fluide selon l'une quelconque des revendications 1 à 8, ladite trousse comprenant :
- un support, par exemple un support de type textile, et
- un échantillon d'au moins un composant enzymatique sous forme solide à mettre en solution avant l'imprégnation du support.

17. Trousse selon l'une ou l'autre des revendications 15 et 16, **caractérisée en ce qu'**elle comprend en outre un échantillon d'au moins un composant détergent.

18. Trousse selon la revendication 17, **caractérisée en ce que** ledit échantillon d'au moins un détergent comprend au moins un agent mouillant et/ou au moins un agent séquestrant et/ou au moins un agent dispersant.

19. Trousse selon l'une quelconque des revendications 15 à 18, **caractérisée en ce que** ledit un échantillon d'au moins un composant enzymatique comprend au moins une protéase et/ou au moins une laccase et/ou au moins une polysaccharidase.

20. Utilisation d'un support selon l'une quelconque des revendications 1 à 8 pour le prélèvement de microorganismes présents sur une surface, en particulier pour le prélèvement de microorganismes ayant formé ou non un biofilm sur ladite surface.

21. Utilisation d'une trousse selon l'une quelconque des revendications 15 à 19 pour le prélèvement de microorganismes présents sur une surface, en particulier pour le prélèvement de microorganismes ayant formé ou non un biofilm sur ladite surface.

## Patentansprüche

1. Träger, der mit mindestens einer flüssigen Zusammensetzung imprägniert ist, die mindestens eine Enzymkomponente in Lösung umfasst, zur Anwendung auf einer Oberfläche, die Mikroorganismen aufweist, die einen Biofilm gebildet haben oder nicht, **dadurch gekennzeichnet, dass** der imprägnierte Träger für eine Probenahme der Mikroorganismen auf der Oberfläche bestimmt ist, und dadurch, dass die mindestens eine Zusammensetzung eine dynamische Viskosität im Bereich zwischen 50 und 2000 mPa.s aufweist.

2. Träger nach Anspruch 1, **dadurch gekennzeichnet, dass** die mindestens eine Enzymkomponente mindestens eine Protease und/oder mindestens eine Laccase und/oder mindestens eine Polysaccharidase umfasst.

3. Träger nach Anspruch 1 oder 2, **dadurch gekennzeichnet, dass** der Träger ein Tuch oder ein Schwamm oder ein Stoff oder jede andere Art von Träger ist, die geeignet ist.

4. Träger nach einem der Ansprüche 1 bis 3, **dadurch gekennzeichnet, dass** die mindestens eine Zusammensetzung, die mindestens eine Enzymkomponente umfasst, einen pH-Wert im Bereich zwischen 5 und 11 aufweist.

5. Träger nach einem der Ansprüche 1 bis 4, **dadurch gekennzeichnet, dass** der Träger und die mindestens eine Zusammensetzung, die mindestens eine Enzymkomponente umfasst, steril sind.

6. Träger nach einem der Ansprüche 1 bis 5, **dadurch gekennzeichnet, dass** die mindestens eine Zusammensetzung, die mindestens eine Enzymkomponente umfasst, weiter mindestens eine Reinigungsmittelkomponente umfasst.

7. Träger nach Anspruch 6, **dadurch gekennzeichnet, dass** die mindestens eine Reinigungsmittelkomponente mindestens ein Benetzungsmittel und/oder mindestens ein Sequestriermittel und/oder mindestens ein Dispergiermittel umfasst.

8. Träger nach den Ansprüchen 6 oder 7, **dadurch gekennzeichnet, dass** die mindestens eine Reinigungsmittelkomponente einen Sequestriermittelanteil im Bereich zwischen 1 und 10 Gew.-%, einen Dispergiermittelanteil im Bereich zwischen 1 und 10 Gew.-%, und einen Benetzungsmittelanteil im Bereich zwischen 1 und 30 Gew.-%, bezogen auf das Gesamtgewicht der mindestens einen Reinigungsmittelkomponente, umfasst.

9. Verfahren zur Probenahme von Mikroorganismen, die auf einer Oberfläche vorhanden sind, insbesondere zur Probenahme von Mikroorganismen, die auf der Oberfläche einen Biofilm gebildet haben oder nicht, mit Hilfe eines mit mindestens einer flüssigen Zusammensetzung imprägnierten Trägers nach einem der Ansprüche 1 bis 8, wobei das Verfahren die folgenden Schritte umfasst:
- Aufbringen des imprägnierten Trägers auf die Oberfläche während einer vorbestimmten Zeitdauer, um von der Oberfläche Mikroorganismen, die dort einen Biofilm gebildet haben oder nicht, zu entnehmen; und
- Entfernen des Trägers von der Oberfläche.

10. Verfahren nach Anspruch 9, **dadurch gekennzeichnet, dass** die vorbestimmte Zeitdauer des Aufbringens des imprägnierten Trägers auf die Oberfläche im Bereich zwischen 1 und 45 Minuten beträgt, und vorzugsweise 15 Minuten beträgt.

11. Verfahren nach den Ansprüchen 9 oder 10, **dadurch gekennzeichnet, dass** es einen vorhergehenden Schritt des Erwärmens der mindestens einen Zusammensetzung, die die mindestens eine Enzymkomponente umfasst, auf eine Temperatur im Bereich zwischen 15 und 65 °C, vorzugsweise auf eine Temperatur von gleich 45 °C, umfasst.

12. Verfahren nach einem der Ansprüche 9 bis 11, **dadurch gekennzeichnet, dass** es einen zusätzlichen Schritt des Abstreichens der Oberfläche derart umfasst, dass Mikroorganismen, die von dem imprägnierten Träger nicht entnommen wurden, mit Hilfe von Abstrichtupfern entnommen werden.

13. Verfahren nach einem der Ansprüche 9 bis 12, **dadurch gekennzeichnet, dass** es einen zusätzlichen Schritt des Extrahierens der entnommenen Mikroorganismen in einer sterilen Extraktionslösung umfasst.

14. Verfahren nach Anspruch 13, **dadurch gekennzeichnet, dass** es einen zusätzlichen Schritt des Quantifizierens und/oder Identifizierens der extrahierten Mikroorganismen umfasst.

15. Set zur Herstellung eines mit mindestens einer flüssigen Zusammensetzung imprägnierten Trägers nach einem der Ansprüche 1 bis 8, wobei das Set umfasst:
- einen Träger, zum Beispiel einen Träger vom textilen Typ; und
- eine Probe mindestens einer Enzymkomponente in Lösung.

16. Set zur Herstellung eines mit mindestens einer flüssigen Zusammensetzung imprägnierten Trägers nach einem der Ansprüche 1 bis 8, wobei das Set umfasst:
- einen Träger, zum Beispiel einen Träger vom textilen Typ, und
- eine Probe mindestens einer Enzymkomponente in fester Form, die vor dem Imprägnieren des Trägers in Lösung gebracht werden muss.

17. Set nach einem der Ansprüche 15 oder 16, **dadurch gekennzeichnet, dass** es weiter eine Probe mindestens einer Reinigungsmittelkomponente umfasst.

18. Set nach Anspruch 17, **dadurch gekennzeichnet, dass** die Probe mindestens eines Reinigungsmittels mindestens ein Benetzungsmittel und/oder mindestens ein Sequestriermittel und/oder mindestens ein Dispergiermittel umfasst.

19. Set nach einem der Ansprüche 15 bis 18, **dadurch gekennzeichnet, dass** die eine Probe mindestens einer Enzymkomponente mindestens eine Protease und/oder mindestens eine Laccase und/oder mindestens eine Polysaccharidase umfasst.

20. Verwendung eines Trägers nach einem der Ansprüche 1 bis 8 zur Probenahme von Mikroorganismen, die auf einer Oberfläche vorhanden sind, insbesondere zur Probenahme von Mikroorganismen, die auf der Oberfläche einen Biofilm gebildet haben oder nicht.

21. Verwendung eines Sets nach einem der Ansprüche 15 bis 19 zur Probenahme von Mikroorganismen, die auf einer Oberfläche vorhanden sind, insbesondere zur Probenahme von Mikroorganismen, die auf der Oberfläche einen Biofilm gebildet haben oder nicht.

## Claims

1. Substrate impregnated with at least one fluid composition comprising at least one enzymatic component in a solution for an application on a surface having microorganisms, having formed or not a biofilm, **characterised in that** said impregnated substrate is intended for a sampling of said microorganisms on said surface, and **in that** said at least one composition has a dynamic viscosity of between 50 and 2000mPa.s.

2. Substrate according to claim 1, **characterised in that** said at least one enzymatic component comprises at least one protease and/or at least one laccase and/or at least one polysaccharidase.

3. Substrate according to claim 1 or 2, **characterised in that** said substrate is a wipe or a sponge or a cloth, or any other type of suitable substrate.

4. Substrate according to any one of claims 1 to 3, **characterised in that** said at least one composition comprising at least one enzymatic component has a pH of between 5 and 11.

5. Substrate according to any one of claims 1 to 4, **characterised in that** said substrate and said at least one composition comprising at least one enzymatic component are sterile.

6. Substrate according to any one of claims 1 to 5, **characterised in that** said at least one composition comprising at least one enzymatic component further comprises at least one detergent component.

7. Substrate according to claim 6, **characterised in that** said at least one detergent component comprises at least one wetting agent and/or at least one sequestering agent and/or at least one dispersing agent.

8. Substrate according to claims 6 or 7, **characterised in that** said at least one detergent component comprises a proportion of sequestering agent of between 1 and 10%, a proportion of dispersing agent of between 1 and 10% and a proportion of wetting agent of between 1 and 30% by weight with respect to the total weight of said at least one detergent component.

9. Method for sampling microorganisms present on a surface, in particular, for sampling microorganisms having formed, or not, a biofilm on said surface, using a substrate impregnated with at least one fluid composition according to any one of claims 1 to 8, said method comprising the following steps:
- an application of said substrate impregnated on said surface for a predetermined time period in order to sample, starting with said surface, microorganisms having formed, or not, a biofilm there; and
- a removal of said substrate from said surface.

10. Method according to claim 9, **characterised in that** said predetermined time period of application of said impregnated substrate on said surface is between 1 and 45 minutes and is preferably 15 minutes.

11. Method according to claims 9 or 10, **characterised in that** it comprises a prior step of heating said at least one composition comprising said at least one enzymatic component at a temperature of between 15 and 65°C, preferably at a temperature equal to 45°C.

12. Method according to any one of claims 9 to 11, **characterised in that** it comprises an additional step of swabbing said surface, such that microorganisms not sampled by said impregnated substrate are sampled using swabs.

13. Method according to any one of claims 9 to 12, **characterised in that** it comprises an additional step of extracting said sampled microorganisms in a sterile extraction solution.

14. Method according to claim 13, **characterised in that** it comprises an additional step of quantifying and/or identifying said extracted microorganisms.

15. Kit for preparing a substrate impregnated with at least one fluid composition according to any one of claims 1 to 8, said kit comprising:
- a substrate, for example, a textile-type substrate; and
- a sample of at least one enzymatic component in solution.

16. Kit for preparing a substrate impregnated with at least one fluid composition according to any one of claims 1 to 8, said kit comprising:
- a substrate, for example a textile-type substrate, and
- a sample of at least one enzymatic component in solid form to be put in solution before the impregnation of the substrate.

17. Kit according to either of claims 15 and 16, **characterised in that** it further comprises a sample of at least one detergent component.

18. Kit according to claim 17, **characterised in that** said sample of at least one detergent comprises at least one wetting agent and/or at least one sequestering agent and/or at least one dispersing agent.

19. Kit according to any one of claims 15 to 18, **characterised in that** said one sample of at least one enzymatic component comprises at least one protease and/or at least one laccase and/or at least one polysaccharidase.

20. Use of a substrate according to any one of claims 1 to 8 for the sampling of microorganisms present on a surface, in particular for the sampling of microorganisms having formed, or not, a biofilm on said surface.

21. Use of a kit according to any one of claims 15 to 19 for the sampling of microorganisms present on a surface, in particular, for the sampling of microorganisms having formed, or not, a biofilm on said surface.
